(19) Europäisches Patentamt
European Patent Office
Office européen des brevets

(11) Veröffentlichungsnummer: **0 014 835**
**B1**

# (12) EUROPÄISCHE PATENTSCHRIFT

(45) Veröffentlichungstag der Patentschrift: **15.07.81**

(51) Int. Cl.³: **C 07 C 43/17,** C 07 C 41/22, C 07 C 47/24, C 07 C 45/42

(21) Anmeldenummer: **80100160.3**

(22) Anmeldetag: **14.01.80**

## (54) Bis-(brom- und chlor-substituiertes propenyl)-äther, Verfahren zu seiner Herstellung und seine Verwendung bei der Herstellung von Trichloracrolein.

(30) Priorität: **24.01.79 JP 6015/79**

(43) Veröffentlichungstag der Anmeldung: **03.09.80 Patentblatt 80/18**

(45) Bekanntmachung des Hinweises auf die Patenterteilung: **15.07.81 Patentblatt 81/28**

(84) Benannte Vertragsstaaten: **BE CH DE FR GB IT NL**

(56) Entgegenhaltungen: **Keine**

(73) Patentinhaber: **NIHON TOKUSHU NOYAKU SEIZO K.K., No.8, 2-chome, Nihonbashi Muromachi, Chuo-Ku, Tokyo (JP)**

(72) Erfinder: **Saito, Junichi, 3-7-12, Osawa, Mitaka-shi, Tokyo (JP)**
Erfinder: **Kume, Toyohiko, 3-18-1, Higashi-Toyoda, Hino-shi, Tokyo (JP)**

(74) Vertreter: **Gremm, Joachim, Dr. et al, Bayer AG c/o Zentralbereich Patente, Marken und Lizenzen Bayerwerk, D-5090 Leverkusen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG.

# Bis-(brom- und chlor-substituiertes propenyl)-äther, Verfahren zu seiner Herstellung und seine Verwendung bei der Herstellung von Trichloracrolein

Die Erfindung betrifft als neue Verbindung den Bis-(1-brom-2,3,3-trichlor-2-propenyl)äther, ein Verfahren zu seiner Herstellung und seine Verwendung als Zwischenprodukt für die Herstellung von Trichloracrolein.

Die Herstellung von Trichloracrolein wurde bereits in der Literatur beschrieben, beispielsweise (A) die Herstellung durch Hydrolyse von 1,1,2,3,3-Pentachlorpropylen (Chemische Berichte 86 (1953), 1469–1476), (B) die Herstellung durch Umsetzung von Butylvinylether, Tetrachlorkohlenstoff und Chlorgas unter geeigneten Reaktionsbedingungen (Bulletin de la Société Chimique de France 1959, 1800–1806), und (C) die Herstellung durch Oxidation von 2,3,3-Trichlor-2-propenylalkohol (vgl. DE-PS 1 285 966).

Alle vorstehend genannten bekannten Verfahren zur Herstellung von Trichloracrolein weisen Nachteile auf, durch die die Verfahren für die grosstechnische Herstellung ungeeignet sind.

Beim vorstehend genannten Verfahren (A) wird eine grosse Menge konzentrierter Schwefelsäure bei der Reaktion verwendet, und die Produktausbeute ist sehr niedrig. Ferner ist das Ausgangsmaterial, 1,1,2,3,3-Pentachlorpropylen, nicht leicht verfügbar.

Das Verfahren (B) umfasst komplizierte Reaktionsfolgen und erfordert sehr hohe Temperaturen, obwohl die Verfügbarkeit der Ausgangsmaterialien und die Ausbeute des Produkts keine Probleme aufwerfen.

Das Verfahren (C) weist die Nachteile auf, dass das nicht umgesetzte Ausgangsmaterial, 2,3,3-Trichlor-2-propenylalkohol, abgetrennt werden muss, und dass das Ausgangsmaterial, der 2,3,3-Trichlor-2-propenylalkohol selbst, nicht leicht verfügbar ist.

Es besteht somit noch ein Bedürfnis für ein grosstechnisch durchführbares und vorteilhaftes Verfahren zur Herstellung von Trichloracrolein, das als Zwischenprodukt für die Herstellung gewisser Farbstoffe, Arzneimittel und landwirtschaftlicher Chemikalien wertvoll ist.

Gegenstand der Erfindung ist der Bis-(1-brom-2,3,3-trichlor-2-propenyl)äther, der eine neue Verbindung ist und die Formel

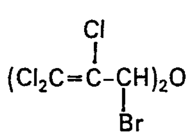

(I)

hat.

Die Erfindung umfasst ferner die Herstellung des Bis(1-brom-2,3,3-trichlor-2-propenyl)äthers der vorstehenden Formel (I) nach einem neuen Verfahren, das dadurch gekennzeichnet ist, dass man den Bis-(2,3,3-trichlor-2-propenyl)äther der Formel

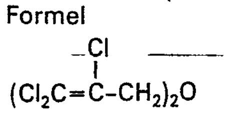

(II)

mit Brom unter Bestrahlung mit Licht (sichtbare Strahlen) umsetzt.

Die Erfindung ist ferner auf die Herstellung von Trichloracrolein der Formel

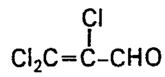

(III)

nach einem Verfahren gerichtet, bei dem man den Bis-(1-brom-2,3,3-trichlor-2-propenyl)äther der vorstehenden Formel (I) der Wasserdampfdestillation in Gegenwart oder Abwesenheit einer anorganischen Säure unterwirft.

Bei diesem Verfahren zur Herstellung von Trichloracrolein kann das Zwischenprodukt (I) in situ gebildet werden. Das Verfahren ist dann dadurch gekennzeichnet, dass man Bis-(2,3,3-trichlor-2-propenyl)äther der Formel (II) mit Brom unter Bestrahlung mit Licht zum Bis-(1-brom-2,3,3-trichlor-2-propenyl)äther der vorstehenden Formel (I) umsetzt und dann den letzteren der Wasserdampfdestillation in Gegenwart oder Abwesenheit einer anorganischen Säure unterwirft.

Der Verlauf der Bromierung des Bis-(2,3,3-trichlor-2-propenyl)äthers ist überraschend, da man die Spaltung der Doppelbindungen unter gleichzeitiger Anlagerung von Bromatomen erwarten würde. Tatsächlich wurde jedoch gefunden, dass entgegen der Erwartung die Wasserstoffatome an den 1-Kohlenstoffatomen des Ethers zuerst angegriffen und durch Bromatome substituiert werden, während die Doppelbindungen keine Reaktion eingehen.

Mit Hilfe der Erfindung kann Trichloracrolein in einer Ausbeute von 85 bis 90% durch einen einfachen Reaktionsprozess hergestellt werden. Ferner weist die in der Zwischenstufe der Reaktion gebildete neue Verbindung, der Bis-(1-brom-2,3,3-trichlor-2-propenyl)äther der Formel (I), biologische Aktivität, insbesondere eine nichttherapeutische bakterizide Wirkung auf. Das Ausgangsmaterial für diese neue Verbindung, der Bis-(2,3,3-trichlor-2-propenyl)-ether, lässt sich leicht aus Trichlorethylen nach einem beschriebenen Verfahren herstellen (US-PS 2 913 500). Trichloracrolein kann vom grosstechnischen Standpunkt leicht und vorteilhaft in allen genannten Fällen hergestellt werden; bei Verwendung von Trichloräthylen als Ausgangsmaterial, bei Verwendung von Bis-(2,3,3-trichlor-2-propenyl)äther als Ausgangsmaterial und bei Verwendung von Bis-(1-brom-2,3,3-trichlor-2-propenyl)äther als Ausgangsmaterial. Es ist auch möglich, Trichloracrolein durch aufeinanderfolgende Reaktionen in guter Ausbeute herzustellen, ohne die neue Verbindung, den Bis-(1-brom-2,3,3-trichlor-2-propenyl)äther der Formel (I), als Zwischenprodukt zu isolieren.

Die Erfindung kann durch das folgende Reaktionsschema veranschaulicht werden:

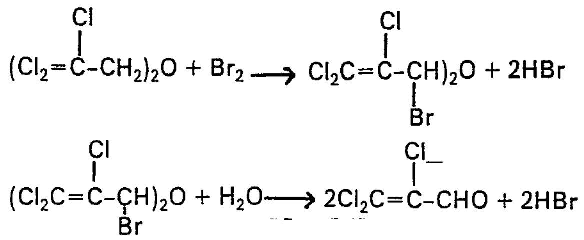

Die Herstellung von Bis-(1-brom-2,3,3-trichlor-2-propenyl)äther wird vorzugsweise unter Verwendung eines Lösungsmittels oder Verdünnungsmittels durchgeführt. Beispiele geeigneter Lösungsmittel und Verdünnungsmittel sind Wasser und inerte organische Lösungsmittel, z.B. aliphatische, alicyclische und aromatische Kohlenwasserstoffe (die gegebenenfalls chloriert sein können), z.B. Hexan, Cyclohexan, Petroläther, Ligroin, Benzol, Methylenchlorid, Chloroform, Tetrachlorkohlenstoffe, Äthylenchlorid, Trichloräthylen und Chlorbenzol.

Die Bromierungsreaktion beim Verfahren gemäss der Erfindung kann innerhalb eines weiten Temperaturbereichs durchgeführt werden. Im allgemeinen wird sie bei einer Temperatur von -20 °C bis zum Siedepunkt des Reaktionsgemisches, vorzugsweise von 0° bis 100 °C durchgeführt. Vorzugsweise wird die Reaktion bei Normaldruck durchgeführt, jedoch könnte auch bei erhöhtem oder vermindertem Druck gearbeitet werden.

Beispiele anorganischer Säuren, die bei der Wasserdampfdestillation des Bis-(1-brom-2,3,3-trichlor-2-propenyl)äthers verwendet werden können, sind Salzsäure, Bromwasserstoffsäure, Salpetersäure und Schwefelsäure.

Die Erfindung wird durch die folgenden Herstellungsbeispiele weiter erläutert.

Beispiel 1

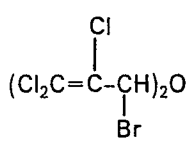
(I)

Zu einer Lösung von 23,9 g Bis-(2,3,3-trichlor-2-propenyl)äther in 50 ml Chloroform wurde tropfenweise eine Lösung von 26,7 g Brom und 20 ml Chloroform gegeben, während mit sichtbarem Licht unter Verwendung von 2 Reflextor-Lampen mit einer Leistung von je 300 Watt bestrahlt wurde. Die Reaktionstemperatur wurde beim Siedepunkt des Chloroforms gehalten, und die Zugabe erfolgte mit einer solchen Geschwindigkeit, dass die Farbe des Broms etwas bestehen blieb. Nach erfolgter Zugabe wurde weiter am Rückfluss erhitzt, um die Reaktion zur Vollendung zu bringen. Nach Beendigung der Reaktion wurde die Chloroformlösung nacheinander mit 3%iger wässriger Natriumbicarbonatlösung und Wasser gewaschen und dann über wasserfreiem Natriumsulfat getrocknet. Das Chloroform wurde abdestilliert und der erhaltene Feststoff aus n-Hexan umkristallisiert, wobei 28,8 g des gewünschten Produkts, d.h. Bis-(1-brom-2,3,3-trichlor-2-propenyl)äther, in Form von farblosen Kristallen vom Schmelzpunkt 95 bis 96 °C erhalten wurden.

Beispiel 2

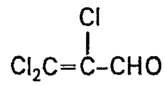
(III)

Zu 23,2 g des gemäss Beispiel 1 hergestellten Bis-(1-brom-2,3,3-trichlor-2-propenyl)äthers wurden 100 ml Wasser und 2 g konzentrierte Schwefelsäure gegeben. Die erhaltene Suspension wurde der Wasserdampfdestillation unterworfen. Der Endpunkt der Wasserdampfdestillation wurde mit wässriger Hydrazinsulfatlösung ermittelt. Das Destillat wurde mit n-Hexan extrahiert und über wasserfreiem Natriumsulfat getrocknet. Das n-Hexan wurde abdestilliert, wobei 13,8 g des gewünschten Produkts, d.h. Trichloracrolein, vom Siedepunkt 101–103 °C/124 mbar erhalten wurden.

Beispiel 3

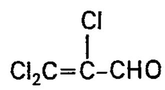
(III)

Zu einer Lösung von 239 g Bis-(2,3,3-trichlor-2-propenyl)äther in 500 ml Chloroform wurde tropfenweise eine Lösung von 267 g Brom und 200 ml Chloroform gegeben, wobei mit sichtbarem Licht unter Verwendung von 2 Reflextor-Lampen mit einer Leistung von je 300 Watt bestrahlt wurde. Die Reaktionstemperatur wurde beim Siedepunkt von Chloroform gehalten, und die Zugabe wurde mit einer solchen Geschwindigkeit vorgenommen, dass die Farbe des Broms etwas bestehen blieb. Nach erfolgter Zugabe wurde weiter bestrahlt und am Rückfluss erhitzt, um die Reaktion zur Vollendung zubringen. Nach Beendigung der Reaktion wurde das Chloroform unter leicht vermindertem Druck abdestilliert. Dem Rückstand wurden 1 l Wasser und 20 g konzentrierte Schwefelsäure zugesetzt, worauf das Gemisch der Wasserdampfdestillation unterworfen wurde. Das Destillat wurde mit n-Hexan extrahiert und über wasserfreiem Natriumsulfat getrocknet. Das n-Hexan wurde abdestilliert, wobei 225 g des gewünschten Produkts, d.h. Trichloracrolein, vom Siedepunkt 101–103 °C/124 mbar erhalten wurden.

Beispiel 4

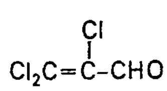
(III)

Der in Beispiel 3 beschriebene Versuch wurde wiederholt, jedoch in einem auf 1/10 verkleinerten Massstab. Nach Beendigung der Reaktion wurde das Chloroform abdestilliert und der Rückstand der Wasserdampfdestillation unterworfen. Das Destillat wurde auf die in Beispiel 2 beschriebene Weise aufgearbeitet, wobei 21 g des gewünschten Produkts, d.h. Trichloracrolein, vom Siedepunkt 101 bis 103 °C/124 mbar erhalten wurde.

**Patentansprüche**

1. Bis-(1-brom-2,3,3-trichlor-2-propenyl)-ether der Formel

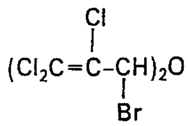 (I)

2. Verfahren zur Herstellung von Bis-(1-brom-2,3,3-trichlor-2-propenyl)äther, dadurch gekennzeichnet, dass man Bis-(2,3,3-trichlor-2-propenyl)äther der Formel

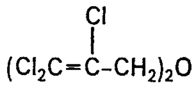 (II)

mit Brom unter Bestrahlung mit Licht umsetzt.

3. Verfahren nach Anspruch 2, dadurch gekennzeichnet, dass man die Reaktion in einem Verdünnungsmittel oder Lösungsmittel durchführt.

4. Verfahren nach Anspruch 3, dadurch gekennzeichnet, dass man als Verdünnungsmittel oder Lösungsmittel Wasser oder einen aliphatischen, alicyclischen oder aromatischen Kohlenwasserstoff, der gegebenenfalls chloriert sein kann, verwendet.

5. Verfahren nach Anspruch 2, 3 oder 4, dadurch gekennzeichnet, dass man die Reaktion bei einer Temperatur von –20 °C bis zum Siedepunkt des Reaktionsgemisches durchführt.

6. Verfahren nach Anspruch 5, dadurch gekennzeichnet, dass man die Reaktion bei einer Temperatur von 0° bis 100 °C durchführt.

7. Verwendung der Verbindung nach Anspruch 1 zur Herstellung von Trichloracrolein.

8. Verfahren zur Herstellung von Trichloracrolein der Formel

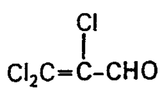 (III),

dadurch gekennzeichnet, dass man den Bis-(1-brom-2,3,3-trichlor-2-propenyl)äther der Formel

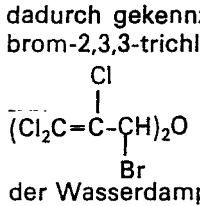 (I)

der Wasserdampfdestillation in Gegenwart oder Abwesenheit einer anorganischen Säure unterwirft.

9. Verfahren nach Anspruch 8, dadurch gekennzeichnet, dass man die Verbindung der Formel (I) in situ herstellt.

10. Verfahren nach Anspruch 8 und 9, dadurch gekennzeichnet, dass man die Wasserdampfdestillation in Gegenwart von Salzsäure, Bromwasserstoffsäure, Salpetersäure oder Schwefelsäure durchführt.

**Claims**

1. Bis-(1-bromo-2,3,3-trichloro-2-propenyl) ether of the formula

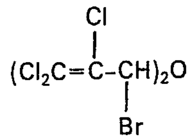 (I).

2. A process for producing bis-(1-bromo-2,3,3-trichloro-2-propenyl) ether, characterised in that bis-(2,3,3-trichloro-2-propenyl) ether, of the formula

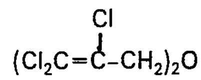 (II),

is reacted with bromine under irradiation with light.

3. A process according to claim 2, characterised in that the reaction is effected in a diluent or solvent.

4. A process according to claim 3, characterised in that the diluent or solvent is water or an aliphatic, alicyclic or aromatic hydrocarbon which may be optionally chlorinated.

5. A process according to claim 2, 3 or 4, characterised in that the reaction is effected at a temperature of from –20 °C to the boiling point of the reaction mixture.

6. A process according to claim 5, characterised in that the reaction is effected at a temperature of from 0° to 100 °C.

7. The use of the compound according to claim 1 for producing trichloroacrolein.

8. A process for producing trichloroacrolein, of the formula

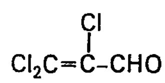 (III),

characterised in that bis-(1-bromo-2,3,3-trichloro-2-propenyl) ether, of the formula

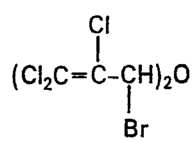 (I),

is steam distilled in the presence or absence of an inorganic acid.

9. A process according to claim 8, characterised in that the compound of the formula (I) is prepared in situ.

10. A process according to claim 8 and 9, characterised in that the steam distillation is effected in the presence of hydrochloric acid, hydrobromic acid, nitric acid or sulphuric acid.

**Revendications**

1. Ether bis-(1-bromo-2,3,3-trichloro-2-propénylique) de formule

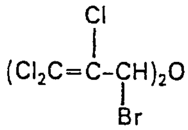
(I)

2. Procédé de préparation de l'éther bis-(1-bromo-2,3,3-trichloro-propénylique), caractérisé en ce que l'on fait réagir l'éther bis-(2,3,3-trichloro-2-propénylique) de formule

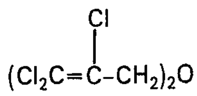
(II)

avec le brome sous irradiation par la lumière.

3. Procédé selon la revendication 2, caractérisé en ce que l'on effectue la réaction dans un diluant ou solvant.

4. Procédé selon la revendication 3, caractérisé en ce que l'on utilise en tant que diluant ou solvant l'eau ou un hydrocarbure aliphatique, alicyclique ou aromatique éventuellement chloré.

5. Procédé selon la revendication 2, 3 ou 4, caractérisé en ce que l'on effectue la réaction à une température comprise entre -20 °C et le point d'ébullition du mélange de réaction.

6. Procédé selon la revendication 5, caractérisé en ce que l'on effectue la réaction à une température de 0 à 100 °C.

7. Utilisation du composé selon la revendication 1 dans la préparation de la trichloracroléine.

8. Procédé de préparation de la trichloracroléine de formule

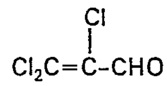
(III)

caractérisé en ce que l'on soumet l'éther bis-(1-bromo-2,3,3-trichloro-2-propénylique) de formule

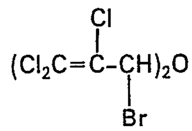
(I)

à la distillation à la vapeur d'eau en présence ou en l'absence d'un acide minéral.

9. Procédé selon la revendication 8, caractérisé en ce que l'on prépare le composé de formule I in situ.

10. Procédé selon les revendications 8 et 9, caractérisé en ce que l'on effectue la distillation à la vapeur d'eau en présence d'acide chlorhydrique, d'acide bromhydrique, d'acide nitrique ou d'acide sulfurique.